# EUROPEAN PATENT SPECIFICATION

(11) **EP 4 021 415 B1**
(45) Date of publication and mention of the grant of the patent: **14.05.2025**
(21) Application number: 20858724.6
(22) Date of filing: 31.08.2020
(51) Int. Cl.: A61K 47/62, A61K 47/69, A61K 9/127, A61K 31/00, C07K 7/06, C07C 69/40, A61P 25/00, A61P 35/00

(54) **CANNABINOID CONTAINING TARGETING LIPOSOMES**
CANNABINOID MIT ZIELGERICHTETEN LIPOSOMEN
LIPOSOMES DE CIBLAGE CONTENANT DES CANNABINOÏDES

(30) Priority: 01.09.2019 US 201962894793 P
(43) Date of publication of application: 06.07.2022
(73) Proprietor: Nextage Therapeutics Ltd., 7403617 Nes Ziona (IL)
(72) Inventor: KLEIN, Joseph Yeshayahu, 3467027 Haifa (IL)
(74) Representative: Schulz Junghans Patentanwälte PartGmbB
(86) International application number: PCT/IL2020/050947
(87) International publication number: WO 2021/038574

(56) References cited:
- WO-A1-01/03668
- WO-A1-2014/076709
- JP-A- H04 295 497
- US-B2- 9 655 848
- APARICIO-BLANCO JUAN ET AL: "Lipid nanocapsules decorated and loaded with cannabidiol as targeted prolonged release carriers for glioma therapy: In vitro screening of critical parameters", EUROPEAN JOURNAL OF PHARMACEUTICS AND BIOPHARMACEUTICS, vol. 134, 22 November 2018 (2018-11-22), pages 126 - 137, XP085555751, ISSN: 0939-6411, DOI: 10.1016/J.EJPB.2018.11.020
- FANNY ASTRUC-DIAZ: "Cannabinoids delivery systems based on supramolecular inclusion complexes and polymeric nanocapsules for treatment of neuropathic pain", HUMAN HEALTH AND PATHOLOGY. UNIVERSITE CLAUDE BERNARD, 23 January 2014 (2014-01-23), Lyon, France, pages 1 - 277, XP055309795, Retrieved from the Internet <URL:https://theses.hal.science/tel-00935588/> [retrieved on 20161012]
- YING M ET AL.: "Liposome-based systemic glioma-targeted drug delivery enabled by all-D peptides", ACS APPLIED MATERIALS & INTERFACES, vol. 8, no. 44, 9 November 2016 (2016-11-09), pages 29977 - 29985, XP055688558
- ZHANG W ET AL.: "Targeting prostate cancer cells with hybrid elastin-like polypeptide/liposome nanoparticles", INTERNATIONAL JOURNAL OF NANOMEDICINE, vol. 13, 9 January 2018 (2018-01-09), pages 293 - 305, XP055794759, Retrieved from the Internet <URL:https://www.ncbi.nlm.nih.gov/pmc/articles/PMC5768422/pdf/ijn-13-293.pdf>
- CHANG HI ET AL.: "Clinical development of liposome-based drugs: formulation, characterization, and therapeutic efficacy", INTERNATIONAL JOURNAL OF NANOMEDICINE, vol. 7, 1 December 2011 (2011-12-01), pages 49 - 60, XP055069345, Retrieved from the Internet <URL:https://www.ncbi.nlm.nih.gov/pmc/articles/PMC3260950/pdf/ijn-7-049.pdf>
- ASTRUC-DIAZ F: "Cannabinoids delivery systems based on supramolecular inclusion complexes and polymeric nanocapsules for treatment of neuropathic pain", DIPLOME DE DOCTORAT, 9 July 2012 (2012-07-09), L’UNIVERSITE CLAUDE BERNARD LYON, pages 111 - 115, XP055309795, Retrieved from the Internet <URL:https://tel.archives-ouvertes.fr/docs/00/93/55/88/PDF/TH2012DiazFanny.pdf>

## Description

### FIELD

Provided herein are liposomes containing a cannabinoid, and a targeting agent.

### BACKGROUND

Blood vessels provide a means in which oxygen and essential nutrients reach cells in different parts of the body. The blood vessels which bring oxygen and nutrients to the central nervous system (CNS), and particularly to the brain, are unique in that various blood components are restricted from passing from the capillaries into the brain. This barrier, which is formed by tight junctions between endothelial cells, forms what is known as the blood-brain barrier in mammals. The blood-brain barrier protects the brain from pathogens and inhibits passage of various solutes and hydrophilic molecules, while allowing passage of oxygen, carbon dioxide and various small molecules, such as glucose into the brain. Some solutes necessary for brain function are actively transported across the blood-brain barrier.

While some pharmaceutical agents may be administered systemically to circulate in the vascular system of a mammal and reach a target tissue or organ, these pharmaceutical agents may be blocked from reaching the brain due to the blood-brain barrier. This makes delivery of pharmaceuticals to the brain, particularly for the treatment of neurological disorders and/or CNS neoplasms difficult.

WO0103668A1 describes liposomal compositions containing a cannabinoid or cannabimimetic agent and methods of systemic delivery by pulmonary administration.

WO2014076709A1 discloses a liposome having a peptide conjugated thereto. The peptide includes a short apolipoprotein E recognition sequence or a short amyloid beta recognition sequence.

### SUMMARY

The invention relates to liposomes containing modified targeting peptides according to claim 1. Liposomes preferably comprise a lipid-based bilayer and at least one cannabinoid.

Also provided herein are methods for making the modified targeting peptides, and the liposomes, and for the use of the liposome according to the invention in treatment of diseases of the CNS according to claim 15.

The foregoing and other objects, features, and advantages will become more apparent from the following detailed description, which proceeds with reference to the accompanying figures.

### BRIEF DESCRIPTION OF THE DRAWINGS

**Fig. 1** is a structural formula of a modified targeting peptide according to an embodiment, showing moieties which it contains;
**Figs 2A-2E** are structural formulae of a modified targeting peptides (compounds 8, 12, 16, 20 and 24) according to an embodiment wherein methionine is in its oxidized form, in the form of methionine sulfoxide, having either ester-amide or ether amide linkers, wherein the n number indicates the number methylene groups between the ester/ ether and the amide group.

### BRIEF DESCRIPTION OF DESCRIBED SEQUENCES

The nucleic and amino acid sequences provided herewith are shown using standard letter abbreviations for nucleotide bases, and three letter code for amino acids, as defined in 37 C.F.R. 1.822. Only one strand of each nucleic acid sequence is shown, but the complementary strand is understood as included by any reference to the displayed strand.

SEQ ID NO: 1 is the amino acid sequence of a targeting peptide, having the sequence: AHRERMS. Optionally, the methionine residue is oxidized.

### DETAILED DESCRIPTION

### I. Terms

Unless otherwise noted, technical terms are used according to conventional usage. Definitions of common terms in molecular biology can be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicates otherwise. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of this disclosure, suitable methods and materials are described below. The term "comprises" means "includes." The abbreviation, *"e.g."* is derived from the Latin *exempli gratia,* and is used herein to indicate a non-limiting example. Thus, the abbreviation "*e.g*." is synonymous with the term "for example."

In case of conflict, the present specification, including explanations of terms, will control. In addition, all the materials, methods, and examples are illustrative and not intended to be limiting.

**Biological Marker:** a measurable, quantifiable indicator which is indicative of a presence of a disease or a medical condition. A biological marker, for example, may be body temperature, an antibody, glucose, or a protein.

**Blood-brain barrier recognition peptide:** a peptide which facilitates the penetration of the liposomal carrier to which the recognition peptide is attached, to the brain from a circulatory system (blood) by binding to a specific transporter located within the blood-brain barrier.

**Cannabinoid:** a compound active on the cannabinoid receptor in a human. Preferably, a phytocannabinoid. A cannabinoid may be produced synthetically, for example, through a chemical synthetic process or by using a biological organism such as a yeast or a bacteria modified to produce the cannabinoid. Alternatively, a cannabinoid may originate from a cannabis plant. A cannabinoid may be isolated, in pure form, or in combination with other cannabinoids.

**Cannabinol** (CBN): a cannabinoid having the structure:

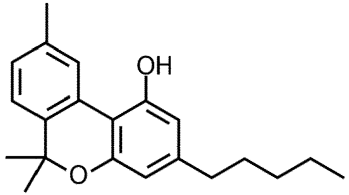

**Cannabis:** a plant from the family Cannabaceae, optionally cannabis sativa, indica and ruderalis. Preferably a plant comprising a cannabinoid.

**CBD:** cannabidiol. A cannabinoid having the structure:

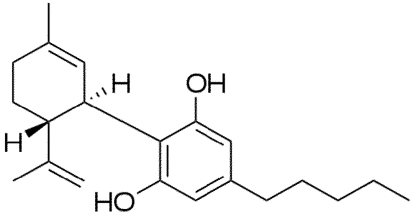

**Liposome:** a composite, generally spherical vesicle comprising a lipid layer or lipid bilayer or multi-layer. Liposomes are typically formed from phospholipids and may be used to encapsulate active pharmaceutical agents or entrap the pharmaceutical agents within the lipid layer.

**THC:** tetrahydrocannabinol. A cannabinoid having the structure:

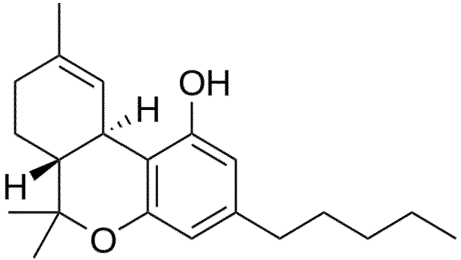

### II. Overview of Several Embodiments

### Modified Targeting Peptides:

Provided herein are modified targeting peptides which can be used to prepare modified targeting peptides and liposomes. According to an embodiment, a modified targeting peptide has a general structure according to Formula [I]: 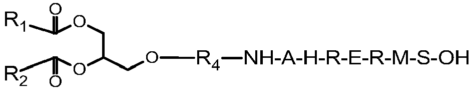 wherein R₁ and R₂ are the same or different and are an alkyl chain having between 13 and 19 carbon atoms, and wherein R₄ is a linker having the structure R₆-R₅-R₇, wherein R₆ and R₇ are each independently a bond or a carbonyl group; and R₅ is selected from the group consisting of: C₁₋₂₀ straight alkyl, C₃₋₂₀ branched alkyl, C₃₋₂₀ cyclic alkyl, and C₆₋₂₀ arylalkyl. Preferably, R₁ and R₂ are a linear (non-branched) alkyl chain having 15 carbon atoms. Preferably, the modified targeting peptide has the structure:

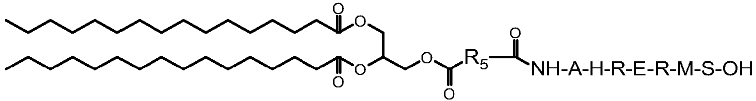

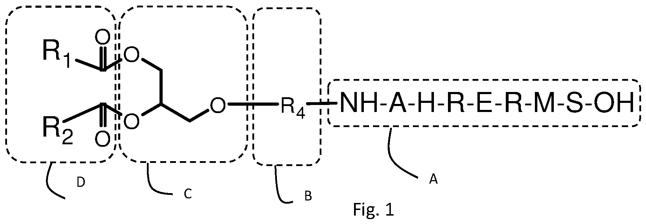

A modified targeting peptide according to an embodiment comprises a structure according to Formula [I] as depicted in Fig. 1. Modified targeting peptide comprises a peptide portion A, a linker portion B, a glycerol moiety C and a fatty acid moiety D. The peptide portion A preferably comprises the peptide having SEQ ID NO: 1, having an oxidized or a non-oxidized methionine, and is bound, via its N-terminus to linker portion B. Linker portion B comprises a linker, as defined by R4, preferably a succinate moiety. Glycerol moiety C is bound to linker moiety B and fatty acid moiety D. Fatty acid moiety D comprises 2 fatty acids, having saturated alkyl chains, wherein R1 and R2 are each alkyl chains having between 13 and 19 carbon atoms, preferably 15 carbon atoms.

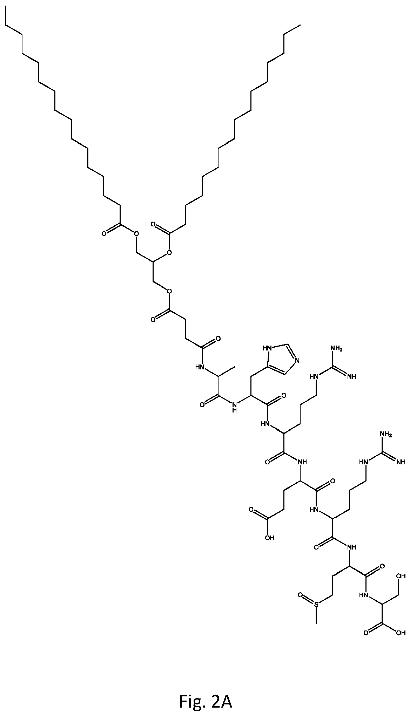

Optionally, the methionine may be present in its oxidized form, in the form of methionine sulfoxide as shown in Figs. 2A-2E.

### Liposomes:

Liposomes comprising the modified targeting peptides can comprise, in addition to the modified targeting peptide, a phospholipid. Preferably, the phospholipid is a phosphatidylcholine. Optionally, the phosphatidylcholine comprises fatty acid groups having between 14 and 20 carbon atoms. Preferably, the phospholipid has a saturated fatty acid moiety. The phospholipid may comprise one or more than one of: 1,2-Dioleoyl-sn-glycero-3-phosphocholine (DOPC); 1,2-Dipalmitoyl-sn-glycero-3-phosphocholine (DPPC); 1,2-Dilauroyl-sn-glycero-3-phosphocholine (DLPC); 1,2-Dimyristoyl-sn-glycero-3-phosphocholine (DMPC); and 1,2-diheptanoyl-sn-glycero-3-phosphocholine (DHPC). The phospholipid is preferably 1,2-distearoyl-sn-glycero-3-phosphocholine (DSPC). Liposomes may also comprise cholesterol.

Liposomes may contain phospholipid and cholesterol, wherein the phospholipid is preferably DSPC in a molar ratio of between 3:1 and 1:1, phospholipid: cholesterol. Preferably, liposomes contain phospholipid and cholesterol, preferably DSPC in a molar ratio of 2:1. Liposomes may contain the modified targeting peptide in a molar percentage of 0.05% to 5%, relative to the content of cholesterol and phospholipid.

### Liposomes may be prepared using the following general method:

A solution of a phospholipid, cannabinoid and modified targeting peptide is prepared. The solution is then combined with a solution of cholesterol. The organic lipid solution is added to the aqueous buffer. The liposome mixture is then extruded using a series of membranes between 400 nanometers (nm) down to 100 nm. This is followed by buffer change and removal of organic solvents and excess cannabinoid not bound by tangential flow filtration (TFF).

According to an embodiment, the phospholipid solution and cholesterol solution are solutions having an alcohol as a solvent. Optionally, the phospholipid solution is a solution in which ethanol is the solvent. Optionally, the cholesterol solution is a solution in which isopropanol is a solvent. Optionally, the buffer solution is an ammonium sulfate buffer. Optionally, the solution is heated to between 50-70°C.

According to an embodiment, the liposomes formed are multilamellar liposomes.

According to an embodiment, the liposome is free of phospholipids having unsaturated fatty acid moieties. According to an embodiment, the liposome is free of 1,2-dioleoyl-sn-glycero-3-phosphocholine (DOPC). According to an embodiment, the liposome is free of 1,2-palmitoyl-phosphatidic acid (DPPA).

The liposome may encapsulate or otherwise bind, by non-covalent bonds, a cannabinoid or a plurality of cannabinoids.

According to an embodiment, the cannabinoid is a hydrophobic cannabinoid.

According to an embodiment, the molar percentage of cannabinoid in the liposome composition is between 0.1% to 50%, preferably 1% to 20% of the total composition. According to an embodiment, the cannabinoid is CBD, CBN, THC, or a combination of 2 or more cannabinoids. According to an embodiment, the cannabinoid is THC. According to an embodiment, the cannabinoid is CBD. According to an embodiment, there are multiple cannabinoids in a single liposome.

Optionally, a plurality of liposomes is provided. In the plurality of liposomes, there is at least one liposome comprising a first cannabinoid and at least one liposome comprising a second cannabinoid.

Other cannabinoids which can be used in conjunction with the liposomes include but not limited to one or a combination of: cannabigerol (CBG), cannabigerolic acid (CBGA), cannabigerol monomethyl ether (CBGM), cannabichromene (CBC), cannabichromanone (CBCN), cannabichromenic acid (CBCA), cannabivarichromene (CBCV), cannabichromevarinic acid (CBCVA), isotetrahydrocannabinol (iso-THC), cannabinol (CBN), cannabinolic acid (CBNA), cannabinol methyl ether (CBNM), cannabinol C₄ (CBN-C₄), cannabinol C₂ (CBN-C₂), cannabinol C₁ (CBN-C₁), cannabinodiol (CBND), cannabielsoin (CBE), cannabielsoic acid A (CBEA-A), Cannabielsoic acid B (CBEA-B), cannabicyclol (CBL), cannabicycloic acid (CBLA), cannabicyclovarin (CBLV), cannabitriol (CBT), cannabitriolvarin (CBTV), ethoxy-cannabitriolvarin (CBTVE), cannabivarin (CBV), cannabinodivarin (CBVD), tetrahydrocannabivarin (THCV), cannabidivarin (CBDV), cannabigerovarin (CBGV), cannabigerovarinic acid (CBGVA), cannabifuran (CBF), dehydrocannabifuran (DCBF), cannabirispol (CBR), tetrahydrocannabinolic acid (THCA) and tetrahydrocannabiphorol (THCP).

Optionally, the cannabinoid is in the form of plant extract. The plant extract may comprise multiple cannabinoids, optionally flavonoids and terpenoids.

According to an embodiment, the liposome's diameter is between 50 nm to 300 nm. In another embodiment, the liposome's diameter is between 50 nm to 200 nm. In another embodiment, the liposome's diameter is between 50 nm to 150 nm. In another embodiment, the liposome's diameter is between 90 nm to 140 nm. In another embodiment, the composition comprises a plurality of liposomes having a mean diameter between 50 nm to 300 nm. In another embodiment, the composition comprises a plurality of liposomes having a mean diameter between 50 nm to 200 nm. In another embodiment, the composition comprises a plurality of liposomes having a mean diameter between 50 nm to 150 nm. In another embodiment, the composition comprises a plurality of liposomes having a mean diameter between 50 nm to 250 nm. In another embodiment, the composition comprises a plurality of liposomes having a mean diameter between 90 nm to 200 nm.

According to an embodiment, the zeta potential of a liposome is from 0 millivolt (mV) to -100 mV. In another embodiment, the zeta potential of a liposome is from -5 mV to -40 mV. In another embodiment, the zeta potential of a liposome is from 0 mV to +100 mV. In another embodiment, the zeta potential of a liposome is from +5 to +40. mV.

### Advantages of Modified Targeting Peptides:

Modified targeting peptides described herein are based on modifications of a peptide having an amino acid sequence of SEQ ID NO: 1. The unique modifications described herein provide advantageous qualities to liposomes formed comprising the described modified targeting peptides. Such enhanced qualities include, but are not limited to, increased stability relative to peptides and/or liposomes described in the prior art. The liposomes tend to maintain size and zeta potential over time. Additional advantages include lack of formation of aggregates over time, and suspension uniformity over time.

SEQ ID NO: 1 has been described in United States Patent 9,655,848,. Without being bound by theory, it is suggested that peptides having SEQ ID NO: 1 act as blood brain barrier recognition peptides which, when incorporated in modified targeting peptides described herein, in liposomes described herein, facilitate penetration of the liposome to the brain from the circulatory system, when administered to a mammal. Preferably, administration of the liposome to the mammal does not harm the integrity of the BBB by modifying its permeability to agents not associated with liposomes. Preferably, modified targeting peptides described herein have little to no immunogenicity when administered to mammals.

Whereas US Patent 9,655,848 describes conjugated short peptides incorporating SEQ ID NO: 1, only unsaturated conjugated short peptides are described, for example, 1,2-dioleoyl-sn-glycero-3-succinate-A-H-R-E-R-M-S-COOH. It has been found that such conjugated short peptides are less stable than modified targeting peptides described herein. Reducing agents may need to be added to such conjugated short peptides having unsaturated peptides, in order to increase stability. On the other hand, modified targeting peptides described herein are stable without addition of reducing agents.

### Methods for Treatment:

Some embodiments relate to a therapeutically effective amount of a liposome as described herein for use in treating a disease . The therapeutically effective amount may be an amount, which upon administration to a patient, ameliorates a symptom associated with the disease or modifies the level of a biological marker associated with the disease in the patient.

According to an embodiment, the liposome is administered through a parenteral route. Optionally, the route of administration is intranasal administration. Optionally, the route is injection, via intravenous, subcutaneous or intramuscular routes.

According to an embodiment, the cannabinoid is administered in a liposome to treat a disease or pathology of the brain. The disease or pathology of the brain may be selected from the group consisting of: trauma, infections, neurodegeneration, movement disorders, autoimmune CNS indications, Stroke, ADHD, autism and addiction.

The liposomes described herein may be used for the treatment of: schizophrenia, psychosis, visual or auditory hallucinations, cognitive and social impairments, THC-associated intoxication, cognitive impairments, drug addiction and dependence, alcohol addiction and dependence, anxiety, posttraumatic stress disorder, panic attacks, depression, mania, bipolar disorder, autism, a neurodevelopmental disorders, obsessive-compulsive disorders (OCD), attention deficit and hyperactivity disorder (ADHD), an eating disorder and Rett syndrome.

According to an embodiment, the liposome may be used for the treatment of a brain disease or pathology. The brain disease or pathology may be selected from the group comprising: acoustic neuroma, acquired brain injury, agenesis corpus callosum, Alzheimer's disease, amyotrophic lateral diseases, aneurysm, aphasia, arteriovenous malformation, batten disease, Behçet's disease, blepharospasm, brain tumor, brain cancer, cerebral lupus, cerebral palsy, cervical dystonia, Charcot-Marie-Tooth disorder, Chiari malformation, chronic inflammatory demyelinated polyneuropathy, coma and persistent vegetative state, concussion, Creutzfedlt-Jakob disease, dementia (non-Alzheimer type), depression, Down syndrome, dysautonomia, dyslexia, dyspraxia, dystonia, encephalitis, epilepsy, essential tremor, Friedreich's ataxia, Gaucher disease, Guillain-Barre syndrome, Huntington's disease, hydrocephalus, intracranial hypertension, leukodystrophy, Meniere's disease, meningitis, meningococcal disease, migraine, motor neuron disease, multiple sclerosis, muscular dystrophy, myasthenia gravis, narcolepsy, Parkinson's disease, peripheral neuropathy, Prader-Willi syndrome, progressive supranuclear palsy, restless legs syndrome, Rett syndrome, Shy Drager syndrome, sleep disorders, spasmodic dysphonia, stroke, subarachnoid haemorrhage, Sydenham's chorea, Tay-Sachs disease, Tourette syndrome, transient ischaemic attack, transverse myelitis, trigeminal neuroalgia, tuberous sclerosis, glioblastoma, neuropathic pain, traumatic brain injury and von-Hippel-Lindau syndrome, neuroimmune disorders, and psychiatric diseases.

According to an embodiment, the amount of liposome administered to a patient in need thereof contains within it the amount of cannabinoid equivalent to a known to be effective or approved dosage of the cannabinoid for a given indication. Optionally, the amount of liposome administered to a patient in need thereof contains within it less than the equivalent amount of the cannabinoid known to be effective or approved for a given indication. Optionally, the amount of liposome administered to a patient in need thereof contains within it between about 0.1% and 50% of the equivalent amount of the cannabinoid known to be effective or approved for a given indication.

The following examples are provided to illustrate certain particular features and/or embodiments. These examples should not be construed to limit the disclosure to the particular features or embodiments described.

### EXAMPLES

### Example 1A:

### Preparation of a Modified Targeting Peptide

A modified targeting peptide wherein R₁ and R₂ are each C₁₅H₃₁, and R₅ is (CH₂)₂, and R₆ and R₇ are each carbonyl, was prepared according to the synthesis described below.

The peptide moiety, according to SEQ ID NO: 1, was prepared in protected form, by Atlantic Peptide. Protected form included modifications to Histidine (trityl protection), Arginine (2,2,4,6,7-Pentamethyldihydrobenzofuran-5-sulfonyl), Glu (tert-Butyl), and Serine (tert-Butyl).

### Step 1

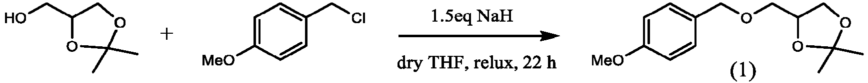

Compound (1) was prepared as described in: Ana Gil-Mesón et al. molecules 2016, 21, 47. Briefly, DL-1,2-isopropylideneglycerol, was added 4-methoxybenzyl chloride in the presence of sodium hydride and potassium iodide in tetrahydrofuran (THF). The reaction mixture was heated at reflux (66 °C), for 16-24 hours (h) to yield crude compound (1) 3-methoxybenzyl-1,2-isopropylideneglycerol.

### Step 2

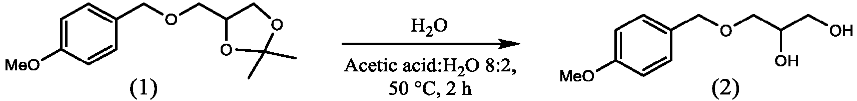

Compound (2) was prepared as described in Masato Abe et al. Biochemistry 2011, 8383. Compound (1) (3-methoxybenzyl-1,2-isopropylideneglycerol), was reacted with acetic acid/water. The reaction mixture was heated for 2h at 50°C to yield crude (2), 1-p-methoxybenzylglycerol.

### Step 3

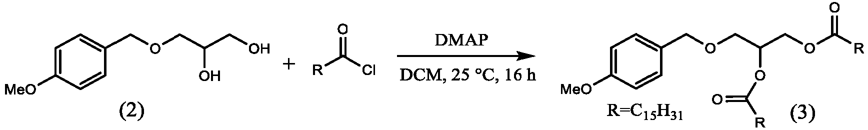

Compound (3) was prepared as described in Masato Abe et al. Biochemistry 2011, 8383. Compound (2), 1-p-methoxybenzylglycerol, palmitoyl chloride, pyridine and 4-(dimethylamino)pyridine (DMAP) in dichloromethane (DCM) were kept at room temperature overnight to yield compound (3), 1-methoxybenzyl-2,3-dipalmitoylglycerol. The overall yield for steps 1-3 was 36%.

### Step 4

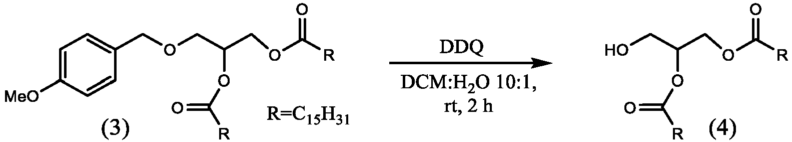

Compound (4) was prepared as described in Stuart, J. Conway et al. Org. Biomol. Chem. 2010, 66. Compound (3) 1-methoxybenzyl-2,3-dipalmitoylglycerol and 2,3-dichloro-5,6-dicyano-p-benzoquinone (DDQ) dichloromethane (DCM)/water were reacted at room temperature to yield compound (4), 1,2-dipalmitoylglycerol, at a yield of 63%.

### Step 5

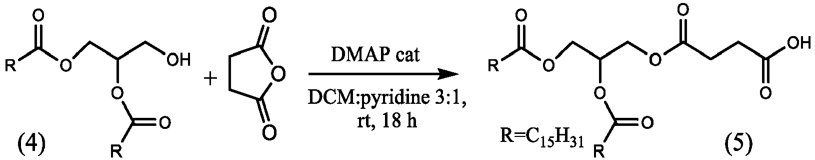

Compound (5) was prepared as described in R. K. Gain et al. Chemistry and Physics of Lipids (1986),237. Compound (4), 1,2-dipalmitoylglycerol, succinic anhydride and 4-(dimethylamino)pyridine were added to pyridine and dichloromethane (DCM). The reaction mixture was kept at room temperatureovernight, to yield compound (5), 1-succinyl-2,3-dipalmitoylglycerol in a yield of 85%.

### Step 6

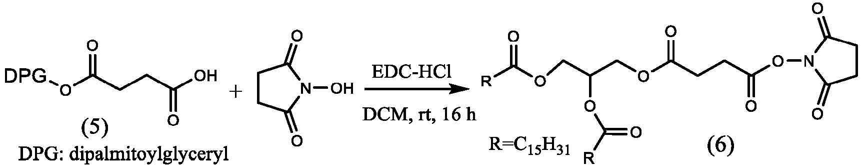

Compound (6) was prepared as described in Ralph Moser et al. J. Org. Chem. (2012), 3143. Compound (5), 1-succinyl-2,3-dipalmitoylglycerol, N-(3-dimethylaminopropyl)-N'-ethylcarbodiimide hydrochloride, (EDC-HCl) and N-hydroxysuccinimide in DCM were stirred at room temperature for overnight, to yield compound (6), 2,3-dipalmitoylglyceryl-1-succinylsuccinimide, in an 89% yield.

### Step 7

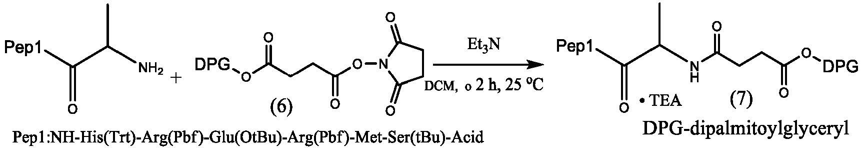

Compound (7) was prepared as described in Unger Evan, C. et al. PCT Application Publication WO 02/36161 A2; 2002. Compound (6), 2,3-dipalmitoylglyceryl-1-succinylsuccinimide, peptide NH2-Ala-His(Trt)-Arg(Pbf)-Glu(OBu)-Arg(Pbf)-Met-Ser(tBu)-OH, and triethylamine in dichloromethane were mixed for 2 h at room temperature to yield compound (7), 2,3-dipalmitoylglyceryl-1-succinyl-NH-Ala-His(Trt)-Arg(Pbf)-Glu(OtBu)-Arg(Pbf)-Met-Ser(tBu)-OH.

### Step 8

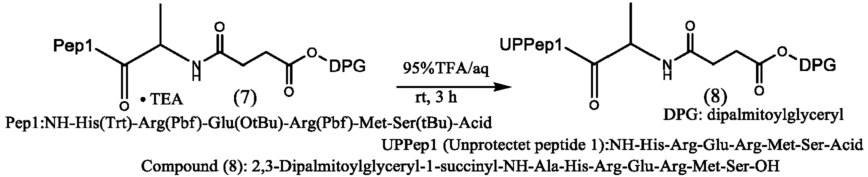

Compound (8) was prepared as described in Tor W. Jensen et al. J. Am. Chem. Soc., 2004, 15223. Compound (7), 2,3-dipalmitoylglyceryl-1-succinyl-NH-Ala-His(Trt)-Arg(Pbf)-Glu(OtBu)-Arg(Pbf)-Met-Ser(tBu)-OH, was treated with trifluoracetic acid (TFA)/water to yield compound (8), 2,3-dipalmitoylglyceryl-1-succinyl-NH-Ala-His-Arg-Glu-Arg-Met-Ser-OH. The yield of steps 7 and 8 was 60%. The methionine residue was partially oxidized. See Fig. 2A.

### Example 1B:

### Preparation of a Modified Targeting Peptide

A modified targeting peptide wherein R₁ and R₂ are each C₁₅H₃₁, R₅ is (CH₂)₃, and R₆ and R₇ are each carbonyl, was prepared according to the synthesis described below. Steps 1-4 were performed as in example 1A.

### Step 5

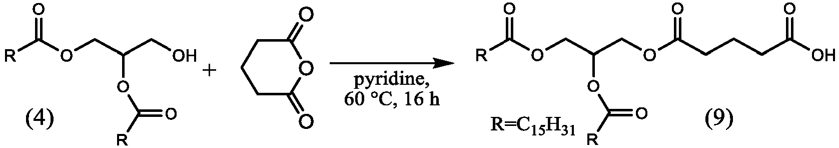

Compound (9) was prepared as described in R. K. Gain et al. Chemistry and Physics of Lipids (1986), 237. Compound (4), 1,2-dipalmitoylglycerol and glutaric anhydride were added to pyridine. The reaction mixture was heated at 60 °C overnight to yield compound (9), 1-glutaryl-2,3-dipalmitoylglycerol at a yield of 64%.

### Step 6

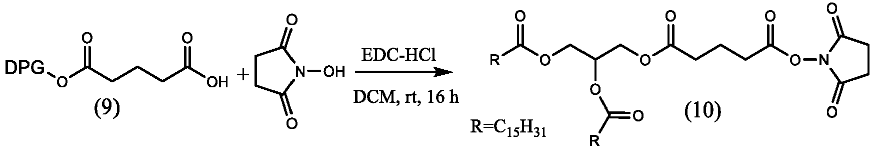

Compound (10) was prepared as described in Ralph Moser et al. J. Org. Chem. (2012), 3143. Compound (9), 1-glutaryl-2,3-dipalmitoylglycerol, N-(3-dimethylaminopropyl)-N'-ethylcarbodiimide hydrochloride, (EDC-HCl) and N-hydroxysuccinimide in dichloromethane (DCM) were stirred at room temperature overnight to yield compound (10), 2,3-dipalmitoylglyceryl-1-glutarylsuccinimide, at a yield of 55%.

### Step 7

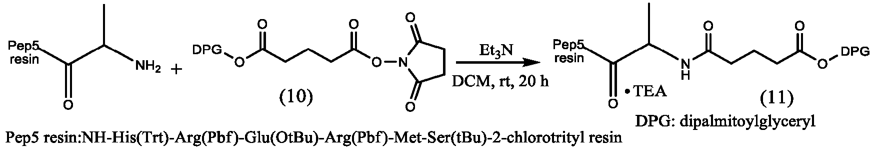

Compound (11) was prepared as described in Tor W. Jensen et al. J. Am. Chem. Soc., 2004, 15223. Compound (10), 2,3-dipalmitoylglyceryl-1-glutarylsuccinimide, peptide NH2-Ala-His(Trt)-Arg(Pbf)-Glu(OBu)-Arg(Pbf)-Met-Ser(tBu)-2-chlorotrityl resin, and triethylamine were mixed in dichloromethane for 16 h at room temperature to yield compound (11), 2,3-dipalmitoylglyceryl-1-glutaryl-NH-Ala-His(Trt)-Arg(Pbf)-Glu(OtBu)-Arg(Pbf)-Met-Ser(tBu)-2-chlorotrityl resin.

### Step 8

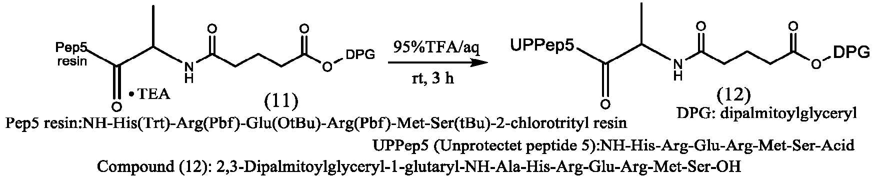

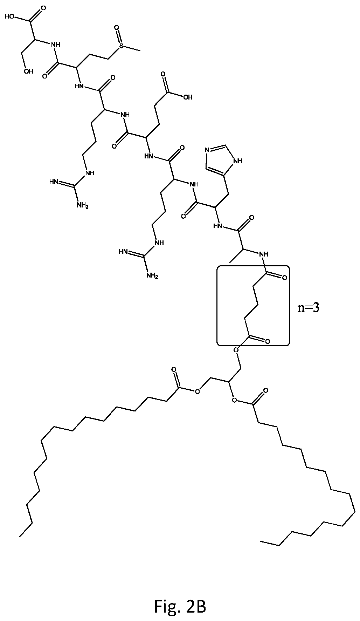

Compound (12) was prepared as described in Tor W. Jensen et al. J. Am. Chem. Soc., 2004, 15223. Compound (11), 2,3-dipalmitoylglyceryl-1-glutaryl-NH-Ala-His(Trt)-Arg(Pbf)-Glu(OtBu)-Arg(Pbf)-Met-Ser(tBu)-2-chlorotrityl resin, was treated with trifluoracetic acid (TFA)/water to yield compound (12), 2,3-dipalmitoylglyceryl-1-glutaryl-NH-Ala-His-Arg-Glu-Arg-Met-Ser-OH. The overall yield of steps 7 and 8 was 24%. The methionine residue was partially oxidized. See Fig. 2B.

### Example 1C:

### Preparation of a Modified Targeting Peptide

A modified targeting peptide wherein R₁ and R₂ are each C₁₅H₃₁, R₅ is CH₂-C(CH₃)₂-CH₂, and R₆ and R₇ are each carbonyl, was prepared according to the synthesis described below. Steps 1-4 were performed as in example 1A.

### Step 5

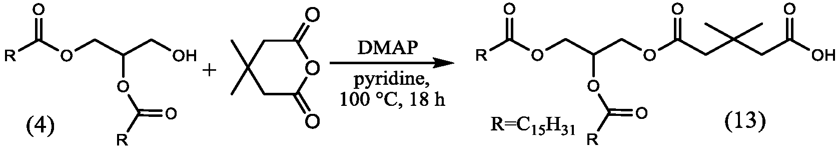

Compound (13) was prepared as described in Sun, I-Chen et al. J. Med. Chem (1998), 41(23), 4648-4657. Compound (4), 1,2-dipalmitoylglycerol, 4-(dimethylamino)pyridine and 3,3-dimethylglutaric anhydride were added to pyridine. The reaction mixture was heated at 100 °C overnight to yield compound (13), 1-(3',3'-dimethylglutaryl)-2,3-dipalmitoylglycerol at a yield of 50%.

### Step 6

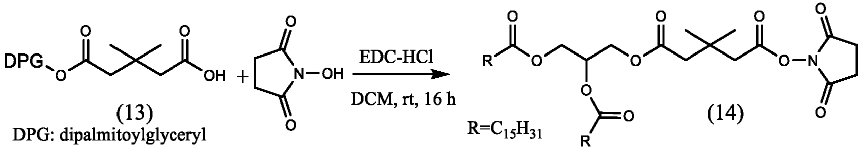

Compound (14) was prepared as described in Ralph Moser et al. J. Org. Chem. (2012), 3143. Compound (13), 1-(3',3'-dimethylglutaryl)-2,3-dipalmitoylglycerol, N-(3-dimethylaminopropyl)-N'-ethylcarbodiimide hydrochloride, (EDC-HCl) and N-hydroxysuccinimide in dichloromethane (DCM) were stirred at room temperature overnight, to yield compound (14), 2,3-dipalmitoylglyceryl-1-(3',3'-dimethylglutaryl)succinimide, at a 32% yield.

### Step 7

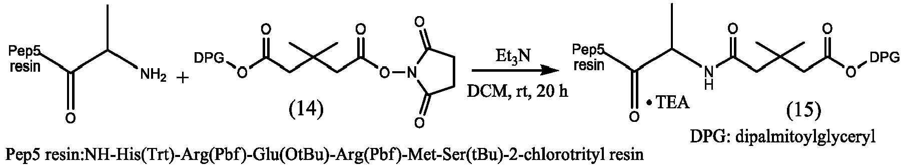

Compound (15) was prepared as described in Tor W. Jensen et al. J. Am. Chem. Soc., 2004, 15223. Compound (14), 2,3-dipalmitoylglyceryl-1-(3',3'-dimethylglutaryl)succinimide, peptide NH2-Ala-His(Trt)-Arg(Pbf)-Glu(OBu)-Arg(Pbf)-Met-Ser(tBu)-2-chlorotrityl resin, and triethylamine were mixed in dichloromethane for 16 h at room temperature to yield compound (15), 2,3-dipalmitoylglyceryl-1-(3',3'-dimethylglutaryl)-NH-Ala-His(Trt)-Arg(Pbf)-Glu(OtBu)-Arg(Pbf)-Met-Ser(tBu)-2-chlorotrityl resin.

### Step 8

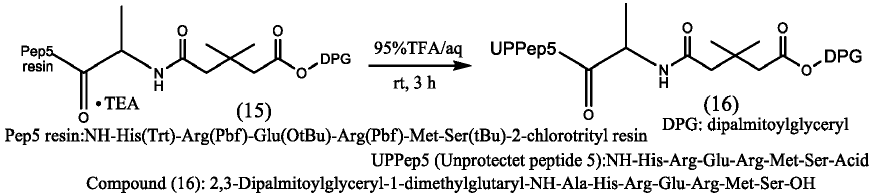

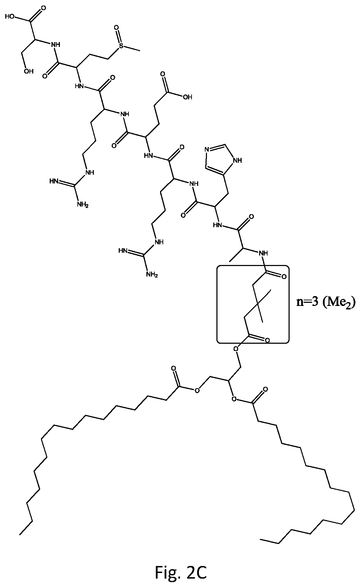

Compound (16) was prepared as described in Tor W. Jensen et al. J. Am. Chem. Soc., 2004, 15223. Compound (15), 2,3-dipalmitoylglyceryl-1-(3',3'-dimethylglutaryl)-NH-Ala-His(Trt)-Arg(Pbf)-Glu(OtBu)-Arg(Pbf)-Met-Ser(tBu)-2-chlorotrityl resin, was treated with trifluoracetic acid (TFA)/water to yield compound (16), 2,3-dipalmitoylglyceryl-1-(3',3'-dimethylglutaryl)-NH-Ala-His-Arg-Glu-Arg-Met-Ser-OH. The overall yield of steps 7 and 8 was 40%. The methionine residue was partially oxidized. See Fig. 2C.

### Example 1D:

### Preparation of a Modified Targeting Peptide

A modified targeting peptide wherein R₁ and R₂ are each C₁₅H₃₁, R₅ is (CH₂)₆, and R₆ and R₇ are each carbonyl, was prepared according to the synthesis described below. Steps 1-4 were performed as in example 1A.

### Step 5

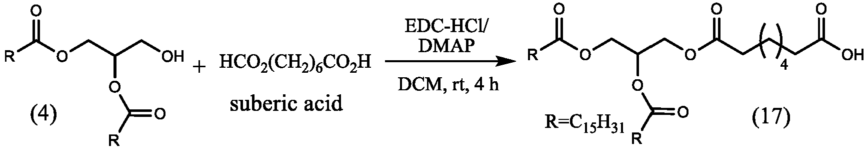

Compound (17) was prepared as described in Baryza, J. L. et al. 2014, WO 2014/136086 Al. Compound (4), 1,2-dipalmitoylglycerol, suberic acid, N-(3-dimethylaminopropyl)-N'-ethylcarbodiimide hydrochloride, (EDC-HCl), and 4-(dimethylamino)pyridine in dichloromethane (DCM) were stirred at room temperature for 4 h to yield compound (17), 1-suberyl-2,3-dipalmitoylglycerol at a yield of 24%.

### Step 6

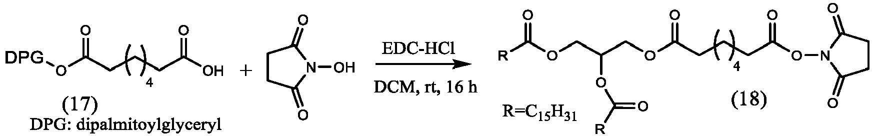

Compound (18) was prepared as described in Ralph Moser et al. J. Org. Chem. (2012), 3143. Compound (17), 1-suberyl-2,3-dipalmitoylglycerol, N-(3-dimethylaminopropyl)-N'-ethylcarbodiimide hydrochloride, (EDC-HCl) and N-hydroxysuccinimide in dichloromethane (DCM) were stirred at room temperature overnight, to yield compound (18), 2,3-dipalmitoylglyceryl-1-suberylsuccinimide, at a 23% yield.

### Step 7

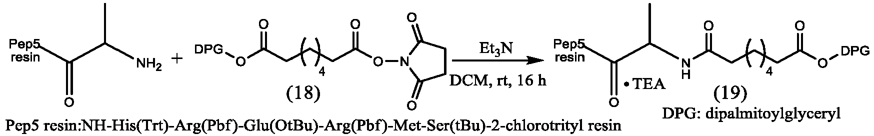

Compound (19) was prepared as described in Tor W. Jensen et al. J. Am. Chem. Soc., 2004, 15223. Compound (18), 2,3-dipalmitoylglyceryl-1-suberylsuccinimide, peptide NH2-Ala-His(Trt)-Arg(Pbf)-Glu(OBu)-Arg(Pbf)-Met-Ser(tBu)-2-chlorotrityl resin, and triethylamine were mixed in dichloromethane for 16 h at room temperature to yield compound (19), 2,3-dipalmitoylglyceryl-1-suberyl-NH-Ala-His(Trt)-Arg(Pbf)-Glu(OtBu)-Arg(Pbf)-Met-Ser(tBu)-2-chlorotrityl resin.

### Step 8

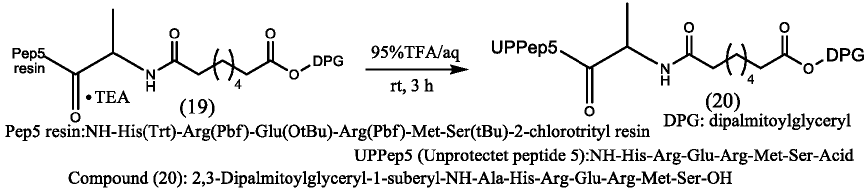

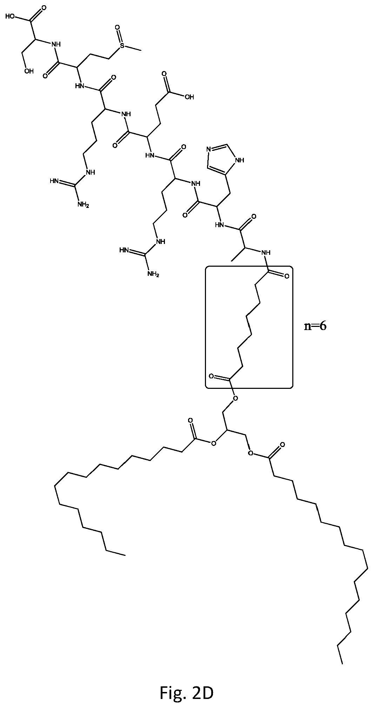

Compound (20) was prepared as described in Tor W. Jensen et al. J. Am. Chem. Soc., 2004, 15223. Compound (19), 2,3-dipalmitoylglyceryl-1-suberyl-NH-Ala-His(Trt)-Arg(Pbf)-Glu(OtBu)-Arg(Pbf)-Met-Ser(tBu)-2-chlorotrityl resin, was treated with trifluoracetic acid (TFA)/water to yield compound (20), 2,3-dipalmitoylglyceryl-1-suberyl-NH-Ala-His-Arg-Glu-Arg-Met-Ser-OH. The overall yield of steps 7 and 8 was 12%. The methionine residue was partially oxidized. See Fig. 2D.

### Example 1E:

### Preparation of a Modified Targeting Peptide

A modified targeting peptide wherein R₁ and R₂ are each C₁₅H₃₁, and R₅ is CH₂, R₆ is a bond and R₇ is carbonyl, was prepared according to the synthesis described below. Steps 1-4 were performed as in example 1A.

### Step 5

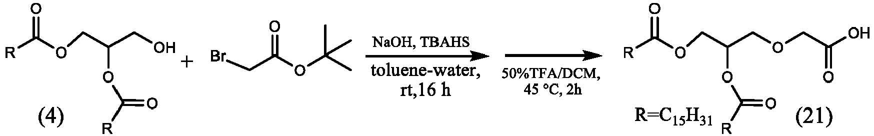

Compound (21) was prepared as described in Baryza, J. L. et al. 2014, WO 2014/136086 Al and as described in Cheaib et al. Tetrahedron: Asymetry (2008), 19(16), 1919-1933. Compound (4), 1,2-dipalmitoylglycerol, tert-butyl bromoacetate, sodium hydroxide, and tetrabutylammonium hydrogensulfate in toluene-water were stirred at room temperature for 16 h to give tert-butyl ester intermediate, which was stirred in TFA/DCM at 45 °C for 2 h to yield compound (21), 1-acetyl-2,3-dipalmitoylglycerol at a yield of 27%.

### Step 6

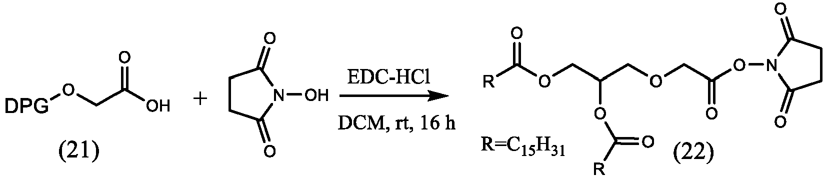

Compound (22) was prepared as described in Ralph Moser et al. J. Org. Chem. (2012), 3143. Compound (21), 1-acetyl-2,3-dipalmitoylglycerol, N-(3-dimethylaminopropyl)-N'-ethylcarbodiimide hydrochloride, (EDC-HCl) and N-hydroxysuccinimide in dichloromethane (DCM) were stirred at room temperature overnight, to yield compound (22), 2,3-dipalmitoylglyceryl-1-acetylsuccinimide, at a 50% yield.

### Step 7

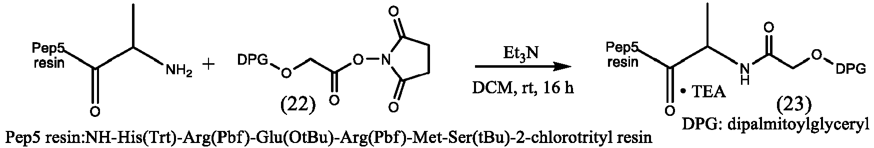

Compound (23) was prepared as described in Tor W. Jensen et al. J. Am. Chem. Soc., 2004, 15223. Compound (22), 2,3-dipalmitoylglyceryl-1-acetylsuccinimide, peptide NH2-Ala-His(Trt)-Arg(Pbf)-Glu(OBu)-Arg(Pbf)-Met-Ser(tBu)-2-chlorotrityl resin, and triethylamine were mixed in dichloromethane for 16 h at room temperature to yield compound (23), 2,3-dipalmitoylglyceryl-1-acetyl-NH-Ala-His(Trt)-Arg(Pbf)-Glu(OtBu)-Arg(Pbf)-Met-Ser(tBu)-2-chlorotrityl resin.

### Step 8

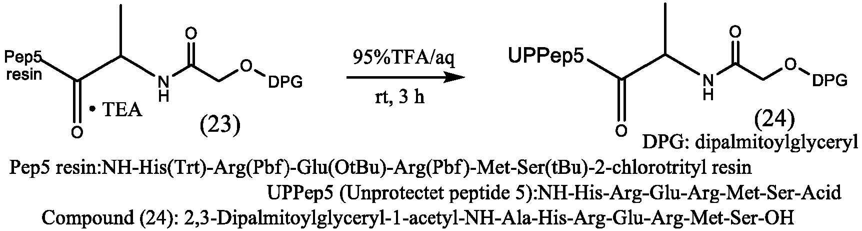

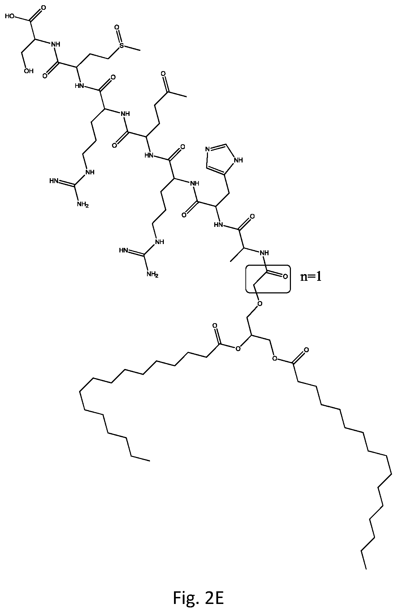

Compound (24) was prepared as described in Tor W. Jensen et al. J. Am. Chem. Soc., 2004, 15223. Compound (23), 2,3-dipalmitoylglyceryl-1-acetyl-NH-Ala-His(Trt)-Arg(Pbf)-Glu(OtBu)-Arg(Pbf)-Met-Ser(tBu)-2-chlorotrityl resin, was treated with trifluoracetic acid (TFA)/water to yield compound (24), 2,3-dipalmitoylglyceryl-1-acetyl-NH-Ala-His-Arg-Glu-Arg-Met-Ser-OH. The overall yield of steps 7 and 8 was 7%. The methionine residue was partially oxidized. See Fig. 2E.

### Example 2:

### Preparation of liposomes encapsulating CBD using modified targeting peptides.

The liposomes were prepared by conventional injection method (S. Batzri and E. D. Korn, Biochim. Biophys. Acta 298, 1015 (1973).

A solution of DSPC, CBD and Compound 8 in ethanol and a solution of Cholesterol in isopropanol were heated in water bath at 60°C with continuous mixing until full dissolution. Both solutions were mixed and rapidly injecting (bolus) to a solution of ammonium sulfate pre-heated to 60°C. The white precipitate of liposome that was formed was extruded at 60°C through membranes of 400nm to 100nm under pressure of 20.7 bar to 6.9 bar.

The resulting liposome solution was subjected to TFF against 0.2M HEPES, 0.15M NaCl buffer, pH= 7.4 to remove the organic solvents and the free small molecules. Liposomes mean particle size were about 100 nm with low polydispersity index (PdI < 0.1) and Zeta potential of -15mV to -40mV.

Some embodiments described herein relate to a liposome comprising a modified peptide having the structure: 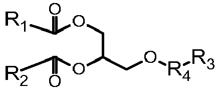
wherein R₁ and R₂ are each the same or different and are an alkyl chain having between 13 and 19 carbon atoms, R₃ is a peptide having a sequence according to SEQ ID NO:1, and wherein R₄ is a linker having the structure R₆-R₅-R₇, wherein R₆ and R₇ are each independently a bond or a carbonyl group; and R₅ is selected from the group consisting of: C₁₋₂₀ straight alkyl, C₃₋₂₀ branched alkyl, C₃₋₂₀ cyclic alkyl, and C₆₋₂₀ arylalkyl; and
at least one cannabinoid. Optionally, R₁ and R₂ are a linear alkyl chain having 15 carbon atoms. Optionally, the peptide is attached to R₄ at the N-terminus of the peptide. Optionally, R₃ comprises the peptide having a sequence according to SEQ ID NO:1 wherein the methionine residue is oxidized in the form of sulfoxide. Optionally, R₆ and R₇ are each a carbonyl group. Optionally, R₆ is a bond and R₇ is a carbonyl group. Optionally, R₅ is a C₁₋₂₀ straight alkyl or a C₃₋₂₀ branched alkyl. Optionally, R₅ is a C₁₋₆ straight alkyl or a C₃₋₆ branched alkyl. Optionally, R₅ is a C₂ straight alkyl. Optionally, R₅ is a CH₂. Optionally, R₅ is a C₃ straight alkyl. Optionally, R₅ is a C₆ straight alkyl. Optionally, R₅ is a CH₂-C(CH₃)₂-CH₂. Optionally, the liposome further comprises a phospholipid and a cholesterol. Optionally, the phospholipid has a saturated fatty acid moiety. Optionally, the phospholipid is 1,2-distearoyl-sn-glycero-3-phosphocholine. Optionally, the molar ratio of phospholipid to cholesterol is between 3:1 and 1:1. Optionally, the molar ratio of phospholipid to cholesterol is 2:1. Optionally, the modified peptide is present in a molar percentage of 0.05% to 5%, relative to the combined amount of cholesterol and phospholipid. Optionally, the modified peptide is present in a molar percentage of 0.5% relative to the combined amount of cholesterol and phospholipid. Optionally, the liposome is free of 1,2-dioleoyl-sn-glycero-3-phosphocholine (DOPC). Optionally, the liposome is free of 1,2-palmitoyl-phosphatidic acid (DPPA). Optionally, the cannabinoid is hydrophobic. Optionally, the cannabinoid is CBD, CBN, or THC. Optionally, the cannabinoid is selected from the group consisting of: cannabigerol (CBG), cannabigerolic acid (CBGA), cannabigerol monomethyl ether (CBGM), cannabichromene (CBC), cannabichromanone (CBCN), cannabichromenic acid (CBCA), cannabivarichromene (CBCV), cannabichromevarinic acid (CBCVA), isotetrahydrocannabinol (iso-THC), cannabinolic acid (CBNA), cannabinol methyl ether (CBNM), cannabinol C₄ (CBN-C₄), cannabinol C₂ (CBN-C₂), cannabinol C₁ (CBN-C₁), cannabinodiol (CBND), cannabielsoin (CBE), cannabielsoic acid A (CBEA-A), Cannabielsoic acid B (CBEA-B), cannabicyclol (CBL), cannabicycloic acid (CBLA), cannabicyclovarin (CBLV), cannabitriol (CBT), cannabitriolvarin (CBTV), ethoxy-cannabitriolvarin (CBTVE), cannabivarin (CBV), cannabinodivarin (CBVD), tetrahydrocannabivarin (THCV), cannabidivarin (CBDV), cannabigerovarin (CBGV), cannabigerovarinic acid (CBGVA), cannabifuran (CBF), dehydrocannabifuran DCBF, cannabirispol (CBR), tetrahydrocannabinolic acid (THCA) and tetrahydrocannabiphorol (THCP). Optionally, the cannabinoid is in the form of plant extract.

Some embodiments relate to a plurality of liposomes as described above. Optionally, the mean diameter is between 50 nm and 300 nm. Optionally, the mean diameter is between 50 nm and 200 nm. Optionally, the mean diameter is between 50 nm and 150 nm. Optionally, the mean diameter is between 90 nm and 200 nm. Optionally, the zeta potential of a liposome is from -10 mV to -120 mV or from +10 mV to +120 mV. Optionally, the zeta potential of a liposome is from -10 mV to -40 mV or from +10 mV to +40 mV.

Some embodiments described herein relate to a plurality of liposomes for use in a method for treatment of a disease comprising administering to a patient in need thereof said plurality of the liposomes. Optionally, the patient suffers from a disease or pathology of the brain. Optionally, the disease or pathology is selected from the group consisting of: schizophrenia, psychosis, visual or auditory hallucinations, cognitive and social impairments, THC-associated intoxication, cognitive impairments, drug addiction and dependence, alcohol addiction and dependence, anxiety, post traumatic stress disorder, panic attacks, depression, mania, bipolar disorder, autism, a neurodevelopmental disorders, obsessive-compulsive disorders (OCD), attention deficit and hyperactivity disorder (ADHD), an eating disorder and Rett syndrome. Optionally, the disease or pathology is selected from the group consisting of: acoustic neuroma, acquired brain injury, agenesis corpus callosum, Alzheimer's disease, amyotrophic lateral diseases, aneurysm, aphasia, arteriovenous malformation, batten disease, Behçet's disease, blepharospasm, brain tumor, brain cancer, cerebral lupus, cerebral palsy, cervical dystonia, Charcot-Marie-Tooth disorder, Chiari malformation, chronic inflammatory demyelinated polyneuropathy, coma and persistent vegetative state, concussion, Creutzfedlt-Jakob disease, dementia (non-Alzheimer type), depression, Down syndrome, dysautonomia, dyslexia, dyspraxia, dystonia, encephalitis, epilepsy, essential tremor, Friedreich's ataxia, Gaucher disease, Guillain-Barre syndrome, Huntington's disease, hydrocephalus, intracranial hypertension, leukodystrophy, Meniere's disease, meningitis, meningococcal disease, migraine, motor neuron disease, multiple sclerosis, muscular dystrophy, myasthenia gravis, narcolepsy, Parkinson's disease, peripheral neuropathy, Prader-Willi syndrome, progressive supranuclear palsy, restless legs syndrome, Rett syndrome, Shy Drager syndrome, sleep disorders, spasmodic dysphonia, stroke, subarachnoid haemorrhage, Sydenham's chorea, Tay-Sachs disease, Tourette syndrome, transient ischaemic attack, transverse myelitis, trigeminal neuroalgia, tuberous sclerosis, glioblastoma, neuropathic pain, traumatic brain injury, von-Hippel-Lindau syndrome, neuroimmune disorders, and psychiatric disorders. Optionally, the molar percentage of cannabinoid in the liposome is between 1-20% of the composition. Optionally, the cannabinoid is encapsulated within the liposome.

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims.

## Claims

1. A liposome comprising a modified peptide having the structure: 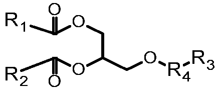
wherein R₁ and R₂ are each the same or different and are an alkyl chain having between 13 and 19 carbon atoms, R₃ is a peptide having a sequence according to SEQ ID NO:1, and wherein R₄ is a linker having the structure R₆-R₅-R₇, wherein R₆ and R₇ are each independently a bond or a carbonyl group; and R₅ is selected from the group consisting of: C₁₋₂₀ straight alkyl, C₃₋₂₀ branched alkyl, C₃₋₂₀ cyclic alkyl, and C₆₋₂₀ arylalkyl; and
at least one cannabinoid.

2. The liposome according to claim 1 wherein R₁ and R₂ are a linear alkyl chain having 15 carbon atoms.

3. The liposome according to claim 1 or 2 wherein the peptide is attached to the succinate moiety at the N-terminus of the peptide.

4. The liposome according to any one of the previous claims wherein R₃ comprises the peptide having a sequence according to SEQ ID NO:1 wherein the methionine residue is oxidized in the form of sulfoxide.

5. The liposome according to any one of the previous claims wherein R₆ and R₇ are each a carbonyl group.

6. The liposome according to any one of the previous claims wherein R₅ is a C₁₋₆ straight alkyl or a C₃₋₆ branched alkyl, preferably a C₂ straight alkyl.

7. The liposome according to any one of the previous claims, further comprising a phospholipid and a cholesterol, wherein the phospholipid is 1,2-distearoyl-sn-glycero-3-phosphocholine.

8. The liposome according to any one of the previous claims wherein the molar ratio of phospholipid to cholesterol is between 3:1 and 1:1, preferably 2:1.

9. The liposome according to any one of the previous claims wherein the modified peptide is present in a molar percentage of 0.05% to 5%, preferably 0.5%, relative to the combined amount of cholesterol and phospholipid.

10. The liposome according to any one of the previous claims, and free of 1,2-dioleoyl-sn-glycero-3-phosphocholine (DOPC) and free of 1,2-palmitoyl-phosphatidic acid (DPPA).

11. The liposome according to any one of the previous claims wherein the cannabinoid is CBD, CBN, THC cannabigerol (CBG), cannabigerolic acid (CBGA), cannabigerol monomethyl ether (CBGM), cannabichromene (CBC), cannabichromanone (CBCN), cannabichromenic acid (CBCA), cannabivarichromene (CBCV), cannabichromevarinic acid (CBCVA), isotetrahydrocannabinol (iso-THC), cannabinolic acid (CBNA), cannabinol methyl ether (CBNM), cannabinol C₄ (CBN-C₄), cannabinol C₂ (CBN-C₂), cannabinol C₁ (CBN-C₁), cannabinodiol (CBND), cannabielsoin (CBE), cannabielsoic acid A (CBEA-A), Cannabielsoic acid B (CBEA-B), cannabicyclol (CBL), cannabicycloic acid (CBLA), cannabicyclovarin (CBLV), cannabitriol (CBT), cannabitriolvarin (CBTV), ethoxy-cannabitriolvarin (CBTVE), cannabivarin (CBV), cannabinodivarin (CBVD), tetrahydrocannabivarin (THCV), cannabidivarin (CBDV), cannabigerovarin (CBGV), cannabigerovarinic acid (CBGVA), cannabifuran (CBF), dehydrocannabifuran DCBF, cannabirispol (CBR), tetrahydrocannabinolic acid (THCA) or tetrahydrocannabiphorol (THCP).

12. The liposome according to any one of the previous claims wherein the cannabinoid is in the form of plant extract.

13. A plurality of liposomes according to any one of the previous claims,wherein the mean diameter is between 50 nm and 300 nm.

14. The plurality of liposomes according to claim 13 wherein the zeta potential of a liposome is from -10 mV to -120 mV or from +10 mV to +120 mV, preferably from -10 mV to -40 mV or from +10 mV to +40 mV.

15. A plurality of liposomes according to any one of claims 13-14 for use in treating a disease or pathology selected from the group consisting of: schizophrenia, psychosis, visual or auditory hallucinations, cognitive and social impairments, THC-associated intoxication, cognitive impairments, drug addiction and dependence, alcohol addiction and dependence, anxiety, post traumatic stress disorder, panic attacks, depression, mania, bipolar disorder, autism, a neurodevelopmental disorders, obsessive-compulsive disorders (OCD), attention deficit and hyperactivity disorder (ADHD), an eating disorder and Rett syndrome, acoustic neuroma, acquired brain injury, agenesis corpus callosum, Alzheimer's disease, amyotrophic lateral diseases, aneurysm, aphasia, arteriovenous malformation, batten disease, Behçet's disease, blepharospasm, brain tumor, brain cancer, cerebral lupus, cerebral palsy, cervical dystonia, Charcot-Marie-Tooth disorder, Chiari malformation, chronic inflammatory demyelinated polyneuropathy, coma and persistent vegetative state, concussion, Creutzfedlt-Jakob disease, dementia (non-Alzheimer type), depression, Down syndrome, dysautonomia, dyslexia, dyspraxia, dystonia, encephalitis, epilepsy, essential tremor, Friedreich's ataxia, Gaucher disease, Guillain-Barre syndrome, Huntington's disease, hydrocephalus, intracranial hypertension, leukodystrophy, Meniere's disease, meningitis, meningococcal disease, migraine, motor neuron disease, multiple sclerosis, muscular dystrophy, myasthenia gravis, narcolepsy, Parkinson's disease, peripheral neuropathy, Prader-Willi syndrome, progressive supranuclear palsy, restless legs syndrome, Rett syndrome, Shy Drager syndrome, sleep disorders, spasmodic dysphonia, stroke, subarachnoid haemorrhage, Sydenham's chorea, Tay-Sachs disease, Tourette syndrome, transient ischaemic attack, transverse myelitis, trigeminal neuroalgia, tuberous sclerosis, glioblastoma, neuropathic pain, traumatic brain injury, von-Hippel-Lindau syndrome, neuroimmune disorders, and psychiatric disorders.

## Patentansprüche

1. Liposom, umfassend ein modifiziertes Peptid mit folgender Struktur: 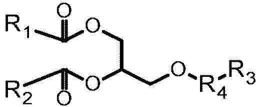
wobei R₁ und R₂ jeweils gleich oder unterschiedlich sind und eine Alkylkette mit zwischen 13 und 19 Kohlenstoffatomen sind, R₃ ein Peptid mit einer Sequenz gemäß SEQ ID NR: 1 ist und wobei R₄ ein Linker mit der Struktur R₆-R₅-R₇ ist, wobei R₆ und R₇ jeweils unabhängig eine Bindung oder eine Carbonylgruppe sind; und R₅ aus der Gruppe ausgewählt ist, die aus geradkettigem C₁₋₂₀-Alkyl, verzweigtkettigem C₃₋₂₀-Alkyl, ringförmigem C₃₋₂₀-Alkyl und C₆₋₂₀-Arylalkyl besteht; und
mindestens ein Cannabinoid.

2. Liposom nach Anspruch 1, wobei R₁ und R₂ eine lineare Alkylkette mit 15 Kohlenstoffatomen sind.

3. Liposom nach Anspruch 1 oder 2, wobei das Peptid an die Succinatgruppe an dem N-Terminus des Peptids angefügt ist.

4. Liposom nach einem der vorangehenden Ansprüche, wobei R₃ das Peptid mit einer Sequenz gemäß SEQ ID NR: 1 umfasst, wobei der Methioninrest in Form von Sulfoxid oxidiert ist.

5. Liposom nach einem der vorangehenden Ansprüche, wobei R₆ und R₇ jeweils eine Carbonylgruppe sind.

6. Liposom nach einem der vorangehenden Ansprüche, wobei R₅ ein geradkettiges C₁₋₆-Alkyl oder ein verzweigtkettiges C₃₋₆-Alkyl, vorzugsweise ein geradkettiges C₂-Alkyl ist.

7. Liposom nach einem der vorangehenden Ansprüche, ferner umfassend ein Phospholipid und ein Cholesterin, wobei das Phospholipid 1,2-Distearoyl-sn-glycero-3-phosphocholin ist.

8. Liposom nach einem der vorangehenden Ansprüche, wobei das Molverhältnis von Phospholipid zu Cholesterin zwischen 3:1 und 1:1, vorzugsweise 2:1, beträgt.

9. Liposom nach einem der vorangehenden Ansprüche, wobei das modifizierte Peptid in einem Stoffmengenanteil von 0,05 % bis 5 %, vorzugsweise 0,5 %, relativ zur kombinierten Menge von Cholesterin und Phospholipid vorliegt.

10. Liposom nach einem der vorangehenden Ansprüche und frei von 1,2-Dioleoyl-sn-glycero-3-phosphocholin (DOPC) und frei von 1,2-Palmitoylphosphatidsäure (DPPA).

11. Liposom nach einem der vorangehenden Ansprüche, wobei das Cannabinoid Folgendes ist: CBD, CBN, THC, Cannabigerol (CBG), Cannabigerolsäure (CBGA), Cannabigerolmonomethylether (CBGM), Cannabichromen (CBC), Cannabichromanon (CBCN), Cannabichromensäure (CBCA), Cannabichromvarin (CBCV), Cannabichromvarinsäure (CBCVA), Isotetrahydrocannabinol (Iso-THC), Cannabinolsäure (CBNA), Cannabinolmethylether (CBNM), Cannabinol-C₄ (CBN-C₄), Cannabinol-C₂ (CBN-C₂), Cannabinol-C₁ (CBN-C₁), Cannabinodiol (CBND), Cannabielsoin (CBE), Cannabielsoinsäure A (CBEA-A), Cannabielsoinsäure B (CBEA-B), Cannabicyclol (CBL), Cannabicyclosäure (CBLA), Cannabicyclovarin (CBLV), Cannabitriol (CBT), Cannabitriolvarin (CBTV), Ethoxy-Cannabitriolvarin (CBTVE), Cannabivarin (CBV), Cannabinodivarin (CBVD), Tetrahydrocannabivarin (THCV), Cannabidivarin (CBDV), Cannabigerovarin (CBGV), Cannabigerovarinsäure (CBGVA), Cannabifuran (CBF), Dehydrocannabifuran (DCBF), Cannabirispol (CBR), Tetrahydrocannabinolsäure (THCA) oder Tetrahydrocannabiphorol (THCP).

12. Liposom nach einem der vorangehenden Ansprüche, wobei das Cannabinoid in Form von Pflanzenextrakt vorliegt.

13. Mehrzahl von Liposomen nach einem der vorangehenden Ansprüche, wobei der mittlere Durchmesser zwischen 50 nm und 300 nm beträgt.

14. Mehrzahl von Liposomen nach Anspruch 13, wobei das Zeta-Potential eines Liposoms -10 mV bis -120 mV oder +10 mV bis +120 mV, vorzugsweise -10 mV bis -40 mV oder +10 mV bis +40 mV, beträgt.

15. Mehrzahl von Liposomen nach einem der Ansprüche 13-14 zur Verwendung bei der Behandlung einer Krankheit oder Krankheitserscheinung, ausgewählt aus der Gruppe, die aus Folgendem besteht: Schizophrenie, Psychose, visuelle oder akustische Halluzinationen, kognitive and soziale Beeinträchtigungen, THC-assoziierte Vergiftung, kognitive Beeinträchtigungen, Drogensucht und -abhängigkeit, Alkoholsucht und -abhängigkeit, Angst, posttraumatische Belastungsstörung, Panikanfälle, Depression, Wahn, bipolare Störung, Autismus, neurologische Entwicklungsstörungen, Zwangsstörungen (OCD), Aufmerksamkeitsdefizit-/Hyperaktivitätsstörung (ADHS), eine Essstörung und Rett-Syndrom, Akustikusneurinom, erworbene Hirnverletzung, Corpus-callosum-Agenesie, Alzheimer-Krankheit, amyotrophe Lateralkrankheiten, Aneurysma, Aphasie, arteriovenöse Missbildung, Batten-Mayou-Krankheit, Behçet-Krankheit, Blepharospasmus, Hirntumor, Hirnkrebs, zerebraler Lupus, zerebrale Lähmung, zervikale Dystonie, Charcot-Marie-Tooth-Störung, Chiari-Missbildung, chronische inflammatorische demyelinisierende Polyneuropathie, Koma und Syndrom reaktionsloser Wachheit, Konkussion, Creutzfeldt-Jakob-Krankheit, Demenz (Nicht-Alzheimer-Typ), Depression, Down-Syndrom, Dysautonomie, Dyslexie, Dyspraxie, Dystonie, Enzephalitis, Epilepsie, essentieller Tremor, Friedreich-Ataxie, Gaucher-Krankheit, Guillain-Barré-Syndrom, Chorea Huntington, Hydrozephalus, intrakranielle Hypertension, Leukodystrophie, Menière-Krankheit, Meningitis, Meningokokken-Krankheit, Migräne, Motorneuronerkrankung, Multiple Sklerose, Muskeldystrophie, Myasthenia gravis, Narkolepsie, Parkinsonsche Krankheit, periphere Neuropathie, Prader-Willi-Syndrom, progressive supranukleäre Blickparese, Restless-Legs-Syndrom, Rett-Syndrom, Shy-Drager-Syndrom, Schlafstörungen, spasmodische Dysphonie, Schlaganfall, Subarachnoidalblutung, Chorea Sydenham, Tay-Sachs-Syndrom, Tourette-Syndrom, transitorische ischämische Attacke, transverse Myelitis, Trigeminusneuralgie, tuberöse Sklerose, Glioblastom, neuropathischer Schmerz, Schädel-Hirn-Trauma, Von-Hippel-Lindau-Syndrom, Neuroimmunstörungen und psychiatrische Störungen.

## Revendications

1. Liposome comprenant un peptide modifié ayant la structure : 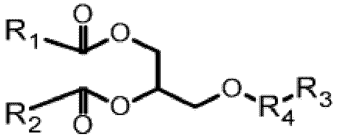
dans laquelle R₁ et R₂ sont chacun identiques ou différents et sont une chaîne alkylique ayant entre 13 et 19 atomes de carbone, R₃ est un peptide ayant une séquence selon SEQ ID N° : 1, et dans laquelle R₄ est un liant ayant la structure R₆-R₅-R₇, dans laquelle R₆ et R₇ sont chacun indépendamment une liaison ou un groupe carbonyle ; et R₅ est sélectionné parmi le groupe constitué de : alkyle linéaire en C₁₋₂₀, alkyle ramifié en C₃₋₂₀, alkyle cyclique en C₃₋₂₀ et arylalkyle en C₆₋₂₀ ; et
au moins un cannabinoïde.

2. Liposome selon la revendication 1, dans lequel R₁ et R₂ sont une chaîne alkylique linéaire ayant 15 atomes de carbone.

3. Liposome selon la revendication 1 ou 2, dans lequel le peptide est relié au groupe fonctionnel de succinate à la terminaison N du peptide.

4. Liposome selon l'une quelconque des revendications précédentes, dans lequel R₃ comprend le peptide ayant une séquence selon SEQ ID N° : 1, dans lequel le résidu de méthionine est oxydé sous la forme de sulfoxyde.

5. Liposome selon l'une quelconque des revendications précédentes, dans lequel R₆ et R₇ sont chacun un groupe carbonyle.

6. Liposome selon l'une quelconque des revendications précédentes, dans lequel R₅ est un alkyle linéaire en C₁₋₆ ou un alkyle ramifié en C₃₋₆, de préférence un alkyle linéaire en C₂.

7. Liposome selon l'une quelconque des revendications précédentes, comprenant en outre un phospholipide et un cholestérol, dans lequel le phospholipide est la 1,2-distéaroyl-sn-glycéro-3-phosphocholine.

8. Liposome selon l'une quelconque des revendications précédentes, dans lequel le rapport molaire du phospholipide sur le cholestérol est compris entre 3/1 et 1/1, de préférence 2/1.

9. Liposome selon l'une quelconque des revendications précédentes, dans lequel le peptide modifié est présent à un pourcentage molaire de 0,05 % à 5 %, de préférence 0,5 %, par rapport à la quantité combinée de cholestérol et de phospholipide.

10. Liposome selon l'une quelconque des revendications précédentes, et exempt de 1,2-dioléoyl-sn-glycéro-3-phosphocholine (DOPC) et exempt d'acide 1,2-palmitoyl-phosphatidique (DPPA).

11. Liposome selon l'une quelconque des revendications précédentes, dans lequel le cannabinoïde est le CBD, le CBN, le cannabigérol de THC (CBG), l'acide cannabigérolique (CBGA), l'éther monométhylique de cannabigérol (CBGM), le cannabichromène (CBC), le cannabichromanone (CBCN), l'acide cannabichroménique (CBCA), le cannabivarichromène (CBCV), l'acide cannabichromévarinique (CBCVA), l'isotétrahydrocannabinol (iso-THC), l'acide cannabinolique (CBNA), l'éther méthylique de cannabinol (CBNM), le cannabinol C₄ (CBN-C₄), le cannabinol C₂ (CBN-C₂), le cannabinol C₁ (CBN-C₁), le cannabinodiol (CBND), le cannabielsoin (CBE), l'acide cannabielsoïque A (CBEA-A), l'acide cannabielsoïque B (CBEA-B), le cannabicyclol (CBL), l'acide cannabicycloïque (CBLA), le cannabicyclovarine (CBLV), le cannabitriol (CBT), le cannabitriolvarine (CBTV), l'éthoxy-cannabitriolvarine (CBTVE), le cannabivarine (CBV), le cannabinodivarine (CBVD), le tétrahydrocannabivarine (THCV), le cannabidivarine (CBDV), le cannabigérovarine (CBGV), l'acide cannabigérovarinique (CBGVA), le cannabifurane (CBF), le déshydrocannabifurane DCBF, le cannabirispol (CBR), l'acide tétrahydrocannabinolique (THCA) ou le tétrahydrocannabiphorol (THCP).

12. Liposome selon l'une quelconque des revendications précédentes, dans lequel le cannabinoïde est sous la forme d'extrait végétal.

13. Pluralité de liposomes selon l'une quelconque des revendications précédentes, dans laquelle le diamètre moyen est compris entre 50 nm et 300 nm.

14. Pluralité de liposomes selon la revendication 13, dans laquelle le potentiel zêta d'un liposome va de -10 mV à -120 mV ou de +10 mV à +120 mV, de préférence de - 10 mV à -40 mV ou de +10 mV à +40 mV.

15. Pluralité de liposomes selon l'une quelconque des revendications 13 et 14 destinée à être utilisée dans le traitement d'une maladie ou pathologie sélectionnée parmi le groupe constitué de : la schizophrénie, la psychose, les hallucinations visuelles ou auditives, les déficiences cognitives et sociales, l'intoxication associée au THC, les déficiences cognitives, l'addiction et la dépendance aux drogues, l'addiction et la dépendance à l'alcool, l'anxiété, le trouble du stress post-traumatique, les attaques de panique, la dépression, la manie, le trouble bipolaire, l'autisme, un trouble neurodéveloppemental, des troubles obsessionnels compulsifs (TOC), le trouble du déficit de l'attention avec hyperactivité (TDAH), un trouble alimentaire et le syndrome de Rett, un neurinome de l'acoustique, une lésion cérébrale acquise, l'agénésie du corps calleux, la maladie d'Alzheimer, les maladies latérales amyotrophiques, l'anévrysme, l'aphasie, une malformation artérioveineuse, la maladie de Batten, la maladie de Behçet, le blépharospasme, une tumeur cérébrale, un cancer cérébral, le lupus cérébral, la paralysie cérébrale, la dystonie cervicale, le trouble de Charcot-Marie-Tooth, la malformation de Chiari, une polyneuropathie démyélinisante inflammatoire chronique, le coma et un état végétatif persistant, la commotion cérébrale, la maladie de Creutzfeldt-Jakob, la démence (de type différent d'Alzheimer), la dépression, la trisomie 21, la dysautonomie, la dyslexie, la dyspraxie, la dystonie, l'encéphalite, l'épilepsie, le tremblement essentiel, l'ataxie de Friedreich, la maladie de Gaucher, le syndrome de Guillain-Barré, la maladie de Huntington, l'hydrocéphalie, l'hypertension intracrânienne, la leucodystrophie, la maladie de Ménière, la méningite, la maladie à méningocoque, la migraine, la maladie neuronale motrice, la sclérose en plaques, la dystrophie musculaire, la myasthénie grave, la narcolepsie, la maladie de Parkinson, la neuropathie périphérique, le syndrome de Prader-Willi, la paralysie supranucléaire progressive, le syndrome des jambes sans repos, le syndrome de Rett, le syndrome de Shy Drager, les troubles du sommeil, la dysphonie spasmodique, l'accident vasculaire cérébral, l'hémorrhagie sous-arachnoïdienne, la chorée de Sydenham, la maladie de Tay-Sachs, le syndrome de Tourette, l'accident ischémique transitoire, la myélite transverse, la névralgie trigéminale, la sclérose tubéreuse, le glioblastome, les douleurs neuropathiques, la lésion cérébrale traumatique, le syndrome de von-Hippel-Lindau, les troubles neuro-immunitaires et les troubles psychiatriques.
